# EUROPEAN PATENT APPLICATION

(11) **EP 1 787 643 A1**
(43) Date of publication of application: **23.05.2007**
(21) Application number: 04774808.2
(22) Date of filing: 09.09.2004
(51) Int. Cl.: A61K 9/52, A61K 9/14, A61K 47/36, A61K 31/573, A61P 27/02

(54) **IMPLANTS AND MICROSPHERES FOR THE SUSTAINED RELEASE OF DRUGS FOR OPHTHALMIC USE AND PREPARATION METHODS THEREOF**

(71) Applicant: Jimenez Bayardo, Arturo, Guadalajara, Jalisco 44290 (MX)
(72) Inventor: QUINTANA HAU, Juan de Dios, C.P. 44290 Guadalajara, Jalisco (MX); TORNERO MONTANO, José Rubén, C.P. 44290 Guadalajara, Jalisco (MX); LOPEZ SANCHEZ, María Isabel, C.P. 44290 Guadalajara, Jalisco (MX); FIGUEROA PONCE, Humberto, C.P. 44290 Guadalajara, Jalisco (MX)
(74) Representative: ABG Patentes, S.L.
(86) International application number: PCT/MX2004/000063
(87) International publication number: WO 2006/028361

(57) **Abstract**

The invention relates to a novel solid complex, which is insoluble in an aqueous environment and which, in dry form thereof, can be used to develop novel systems for the sustained release of drugs for ophthalmic use. The invention also relates to a method of preparing said complex. The complex is formulated based on a viscoelastic solution derived from HA (Biovisc^{®}), a therapeutically acceptable drug such as a steroid, quinolone, a non-steroid anti-inflammatory, an immunosuppressor or compatible drug and chitosan, all of which are conjugated such as to produce biodegradable polymer matrices for the treatment of ocular ailments. The invention also relates to a method of preparing the aforementioned matrices. The invention further relates to microspheres and a method of producing same, using the same original materials, in which the therapeutic principle comprises a steroid, quinolone, a non-steroid anti-inflammatory, an immunosuppressor or another drug which is stable and compatible with said system.

## Description

### 1. Field of the Invention

This invention relates to the composition of implants and microspheres that act as drug release systems for the ocular treatment and a method for the preparation of them.

### 2. Prior Art.

In the area of ophthalmology, distinct presentations, such as suspensions, solutions, emulsions and ointments for the treatment of illnesses have been used for many years, which may be applied locally. It has been observed that the bioavailability of the active ingredient depends, to a great extent, on the type of vehicle and application of a medicine. One of such limitations is the contact time of the medicine with the ocular surface, because the eye's lubrication mechanism, that is blink and lacrimation of the eye, prevents the medicine from remaining sufficient time to achieve a good diffusion of the active ingredient through the conjunctiva or corneal epithelium.

Distinct studies have demonstrated that approximately 1.0% of the drug being applied locally to the eye, crosses the corneal epithelium thus performing its pharmacological action. The challenge of the pharmaceutical industry in the area of ophthalmology has been to develop technology to increase the bioavailability and residence time of a drug. The use of biodegradable polymers for medical uses has been growing rapidly. Polymers have applications in distinct fields of medicine such as tissue engineering (implants or artificial organs), ophthalmology, odontology, traumatology, and other medical fields.

Currently, a series of natural or artificial polymers are known, which have distinct characteristics such as porosity, resistance and susceptibility to being degraded, which have been used for distinct applications. Implants that are made based on biodegradable polymers are an alternative for the treatment of illnesses, being the only alternative widely accepted by the scientific medical community. Implants are used as drug release systems and they are placed in distinct parts of the anatomy of the human body, with the advantage of acting on the location site thereof.

In the area of ophthalmology, a series of different polymer implants have been developed from distinct origin. One of the most widely studied polymeric implants for ophthalmology is poly(DL-lactide) of which its association feasibility with a steroid has been evaluated. The results have indicated compatibility and absence of toxicity at the site of action. Consequently, therapeutic doses are reached in the surrounding areas of the implant and the collateral risks are reduced.

Alternatively, hyaluronic acid or its sodic salt, sodium hyaluronate (HA), has been used as a base for intravitreal implants where their biocompatibility and biodegradation was tested on rabbits. Signs of inflammation were not observed and good compatibility was observed. Other examples of use of intraocular implants are claimed in Canadian patent No. 2,355,313, which describes the use of an implant based on polytetrafluoroethylene, which is biocompatible with intraocular use as a drug release system. Such implants cover the drug with a polymeric layer, which is impermeable to environmental fluids. The drug is delivered by an orifice in the external layer. Such orifice has an area of less than 10% of the total surface area of the implant and drugs of low solubility and high molecular weight may be released therethrough.

Several patents describe the use of different materials for the generation of implants of ophthalmic use. For example, European patent 0488401 describes the use of an intraocular implant for the posterior chamber of the eye following surgical intervention for retina and vitreous disorders. The implant is produced by homogeneously distributing a therapeutic agent associated with the polyactic acid with a particular form. The implant is elaborated by three methods: by addition of solvents, by pressure and heat pressure, achieving a relation of 40-60% of the active ingredient with respect to the total weight of the implant.

U.S. patent No. 4,853,224 describes the way by which ocular implants give a better therapeutic response before any kind of illness. The implants may be prepared based on polysaccharides such as calcium alginate or cellulose, and also based on some polyesters, lactic acid, glycolic acid and polycaprolactone, among others.

U.S. patent No. 5,164,188 describes the use of ocular implants placed in a defined area of the eye. The implant is generally a capsule made of a biodegradable polymer, which may be placed in the suprachoroidea area without the existence of any type of migration. Other polymers used for this purpose are: gelatin and silicon.

In turn, U.S. patent No. 4,863,457 describes a biodegradable inert implant, impregnated with one or more therapeutic agents designed to provide a local and sustained, controlled release of the therapeutic agents.

U.S. patent No. 5,888,533 relates to a method of forming an implant *in situ* within a body, by using non-polymeric materials, and the use of such implants as a drug release system.

### SUMMARY OF THE INVENTION

The main object of the invention is to provide a new formulation of matrices and microspheres consisting of biodegradable polymers of the HA, such as a viscoelastic solution, hereinafter named Biovisc^{®}, associated with a drug and combined with chitosan to be used as a raw material in the design of implants and microspheres of sustained release of drugs for ophthalmic use.

For the effects of this description, in this document, the term "matrix" refers to the ternary association between Biovisc^{®}, chitosan and a drug following the elaboration thereof by a specific process described hereinafter. In turn, the term "implant" refers to a specific geometric form and weight used as a raw material in the matrix. While, the term "microsphere" refers to particles having a specific diameter calculated by the KS300 software (Carl Zeiss), which particles result from a process using Biovisc^{®}, chitosan and a drug.

Furthermore, this invention includes the proposal of methods for the preparation of biodegradable polymeric matrices and microspheres made of a solution deriving from Biovisc^{®}, which presents specific physicochemical properties, and that is associated with an anti-inflammatory steroid or any other drug compatible with the proposed system, and subsequently combined with the chitosan to obtain a triple complex, for its application in ophthalmology.

In the preferred embodiments of the invention, the matrix and microspheres are made up of three main ingredients: a Biovisc^{®} solution, especially formulated from HA, a drug and chitosan.

### BRIEF DESCRIPTION OF DRAWINGS

Figure 1 is a photographic representation of a triangular implant prototype for ophthalmologic use for subconjunctive use, having a length of no more than 1 mm.
Figure 2 is also photographic representation of a circular implant prototype for ophthalmologic use, having a diameter of 5.0 mm for external ocular use.
Figure 3 is a graph representing the kinetics of release of dexamethasone related to a matrix made up of a derivative of Biovisc^{®} and chitosan.
Figure 4 illustrates a statistical analysis of the size of particles based on a sample of liofilized microspheres made of chitosan-Biovisc^{®}-dexamethasone.

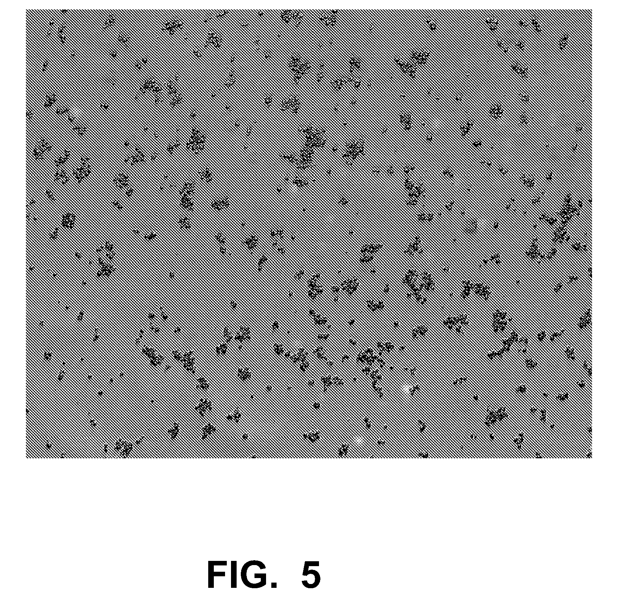

Figure 5 is a photographic view of microspheres made up of chitosan, Biovisc^{®} and dexamethasone, prepared by the method of this invention and then liofilized.
Figure 6 shows the kinetics of release of dexamethasone based on the microspheres made by the corresponding method, wherein the experimental conditions are the same as for the matrices.

### DETAILED DESCRIPTION OF THE PREFERRED EMBODIMENTS OF THE INVENTION

Prior to the detailed description of the characteristics of the invention, hereunder are listed the main elements involved in the composition of the matrixes of microspheres, and their particular characteristics are described that have made them ideal for the development of this new technology with application in the ophthalmologic industry.

### 1. BIOVISC^{®}

The HA or its sodic salt (hyaluronate) is a lineal polymer made up of monomers: N-acetyl glucosamine and glucoronic acid.

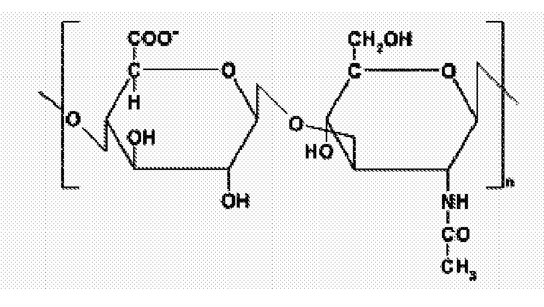

Chemical structure of glucoronic acid dimer and N-acetyl-D-glucosamina. The dimer is repeated "n" times in order to attain the polymer named hyaluronic acid (HA). The addition of sodium chloride to a solution of hyaluronic acid results in the derivative salt known as hyaluronate.

This polymer is a polysaccharide of high molecular weight, which was discovered by Meyer and Palmer in 1934 in the vitreous humor of bovines. The hyaluronate belongs to the group of polysaccharides known as connective tissue polysaccharides, mucopolysaccharides or glucosaminoglycans. To this group belongs: dermatan sulfate, keratan sulfate, heparin sulfate and heparin. Apart from being found in the vitreous humor, hyaluronate is also present in the extracellular matrix and in the synovial fluid. HA may be obtained from distinct sources, such as the cockerel crest, the vitreous humor of fish, the umbilical chord, bacteria and biotechnology. HA is very useful in the pharmaceutical industry because the HA solutions present rheological properties to be deemed as a viscoelastic solution.

In ophthalmology, solutions of distinct concentrations of HA have been used as surgical material during cataract surgery. This has been the basis for the formulation of a new viscoelastic solution named Biovisc^{®} that is made up of an HA solution of 1.6%, sodium chloride at 0.88%, dibasic phosphate of anhydrous sodium at 0.058%, monobasic phosphate of monohydrated sodium at 0.0126% and sterile water for injection as a base for 100%, with a dynamic viscosity of 35,000 ± 5,000 mPas, an osmolarity between 200 and 400 mOsm and a pH between 6.8 and 7.6

### 2. CHITOSAN

Another component of the new formula is chitosan. After cellulose, chitosan is the most abundant polysaccharide found in nature. The carbohydrate is made up of repeated units of β-1,4 N-acetyl glucosamine with a high level of acetylation in the amino group and it is a structural component of the exoskeleton of crustaceans and insects. The controlled treatment of chitin with sodium hydroxide leads to derivatives that differ in level of desacetylation. This modification enables the resulting polymer to be soluble at different levels in weak acids.

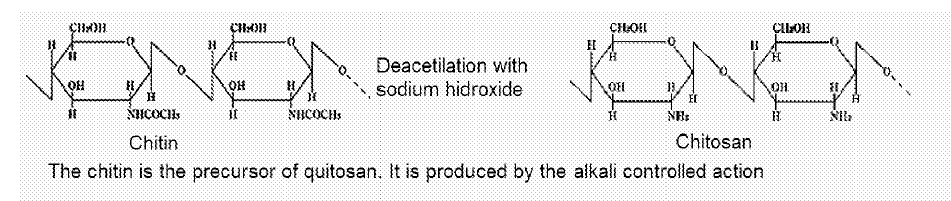

Chitosan shows important biological properties such as the absence of toxicity, biocompatibility and biodegradation in biological systems and at physiological pH is a polycation. The above has been important in order to be considered, in the pharmaceutical industry, as an excipient in different formulations. Furthermore, chitosan has been found to have anti-acid and antiulcer properties, and properties of a hypocholesterolemic action. The level of deacetylation and cationic nature of the molecule makes it a candidate to be used in conjunction with other molecules, such as HA and derivatives combined with other drugs.

### 3. DRUG (DEXAMETHASONE)

In the embodiments of the invention described in this application, the drug used as an example in the formulation of the new matrix for ophthalmic implants, is dexamethasone, which belongs to the group of corticosteroids. As other hormone steroids, dexamethasone acts by modulating the genic expression, and, therefore, on the synthesis of proteins. The main effect of this drug is owed to a strong alteration of the limphocytary immune response, characterized by its anti-inflammatory and immunosuppressant action, which assists the prevention of the other inflammatory processes, including mechanical, chemical and infectious effects. Due to its anti-inflammatory action, dexamethasone is one of the medicines of choice for the treatment of ocular ailments, although its sustained use leads of secondary effects of consideration.

Regarding the invention, the new matrix of biodegradable polymers is characterized in that it takes advantage of the rheological properties of the HA of high molecular weight, which has been submitted to a special treatment and whose resulting derivative, Biovisc^{®}, has the aforementioned characteristics, thereby the homogenous incorporation of aqueous solutions of different drugs therein is possible.

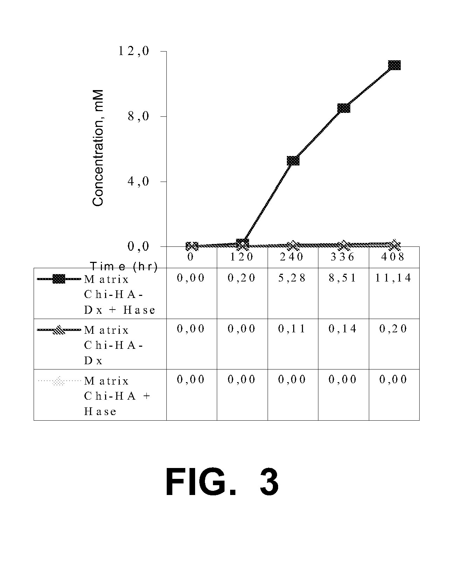

The sensibility of the matrix to the hyalorunidase of bovine and the release of dexamethasone was evaluated *in vitro.* The process was carried out by hydrolyzing 20 mg of the liofilized material, which was hydrated in a solution of sodium chloride and phosphates pH 7.0 (PBS). 100 units of bovine hyalorunidase were added and it was incubated at 37°C. An aliquot of 100 µL was taken, which represented the zero time, after which samples of the same volume were taken at different times. The curves for the witness and the matrices associated with dexamethasone were performed at the same time with and without hyalorunidase. The concentration of dexamethasone was evaluated by of High Performance Liquid Chromatography (HPLC). The results of said evaluation are shown in figure 3.

The controls are defined as matrices created in the same manner, but without the addition of dexamethasone. The data show the sustained liberation of dexamethasone up to more than 408 hours. For the witness, instead of dexamethasone phosphate, a phosphate damper with a pH of 7.0 was used, together with a molar concentration equal to that used for the drug.

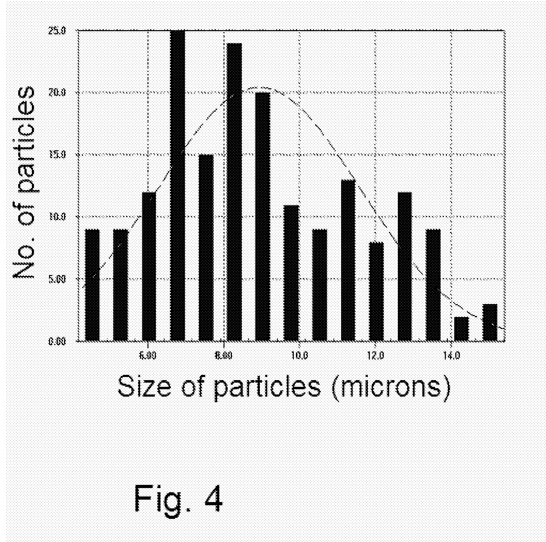

On the other hand, the resulting microspheres have an average diameter below 10 microns, evaluated with KS300 and a Zeiss Axioskop 40 microscope. Figure 4 represents said evaluation.

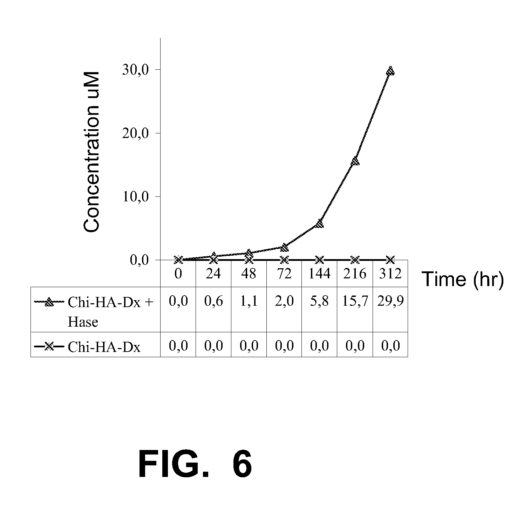

The amount of dexamethasone in the matrices was analyzed in the same manner; an exact amount of the liofilized microspheres were used in order to determine their performance with respect to the action of the bovine hyalorunidase at different times. The performance is shown in figure 6, where a release of dexamethasone in a range of 30 µM is shown up to 312 hours later.

Regarding the method of preparing of Biovisc^{®}, chitosan and dexamethasone matrices, an exemplary method is described here below:
1. diluting exact volume of the solution deriving from HA at 1.6% (Biovisc^{®}) in bidistilled water to obtain a final concentration of 0.025%;
2. adding to the above diluted solution, the exact amount of a solution of 100 mM of dexamethasone phosphate at a final concentration of 30 mM;
3. mixing to homogeneity with a Caframo^{®} agitator using a double-blade propeller inverted at 45° with a diameter of 5 cm;
4. adjusting the pH, if necessary, to about 4.8 by using hydrochloric acid 0.1 N;
5. agitating for approximately 1 hour at 100 revolutions per minute (rpm).
6. adding the exact amount of a solution of chitosan of crab shells at 2% dissolved in acetic acid to obtain a final concentration of 0.8%;
7. agitating for approximately 1 hour all the components added in the above steps, as specified in point 5, until a gel-like insoluble solution is formed.
8. transferring the material to bottles and centrifuge in a Beckham-Coulter centrifuge using a JA14 rotor, at 1,500 rpm for 15 minutes;
9. eliminating the supernatant and rinse the solid sediment with PBS;
10. washing three times with the same buffer in order to eliminate the free dexamethasone phosphate;
11. transferring the wet solid matter to a wide-neck bottle and maintain at -40°C for at least 12 hours; and
12. liofilizing in a Labconco apparatus at 1.33 x 10⁻¹ millibars and -46°C for 72 hours.

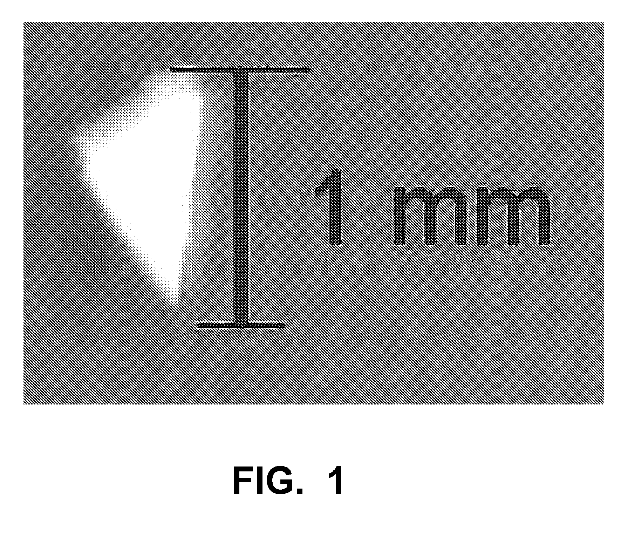

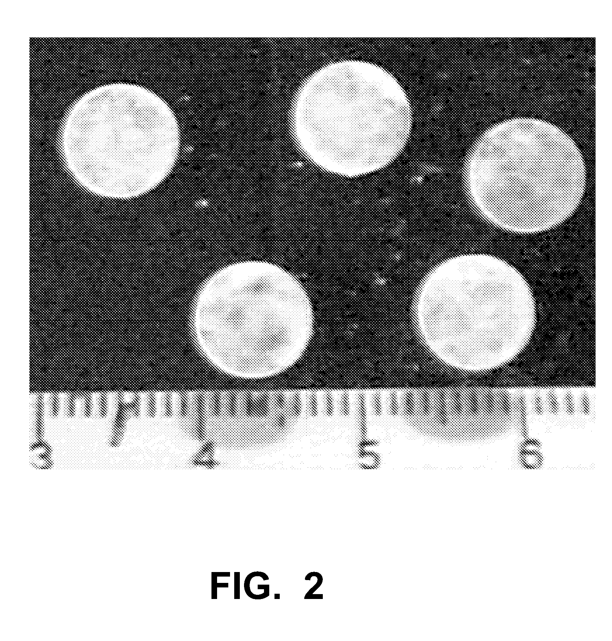

The result of the aforementioned process is a white colored material containing at least 10% of dexamethasone in dry base. The material is stable at room temperature with a relative humidity no more than 25%. This material was used for the preparation of triangular implants for their subconjunctive use, and circular implants for external use. Such implants are illustrated in figures 1 and 2, respectively.

In turn, an exemplary method or process for the preparation of microspheres, is described hereunder:
1. mixing, in a first recipient, 6.25 mL of the solution described as Biovisc^{®}, with 12 mL of dexamethasone phosphate 100 mM for approximately 15 minutes in a Caframo^{®} agitator and a double-blade propeller inverted at 45° with a diameter of 5 cm, at 150 rpm until homogeneity is achieved;
2. mixing, in a second recipient, 74.4 mL of liquid paraffin (mineral oil) with 1.6 mL of Span 80, for approximately 15 minutes in a Caframo^{®} agitator and a double-blade propeller inverted at 45° with a diameter of 5 cm, at 150 rpm until homogeneity is achieved;
3. slowly pouring the content of the first recipient into the second recipient, while maintaining the same agitation speed, until both contents are incorporated, and increase the centrifuging speed to 1,300 rpm and agitate for at least 12 hours;
4. adding 21.75 mL of chitosan of crab shells at 1.47% until the full incorporation thereof into the system;
5. agitating in the conditions specified in step 3, for 24 hours;
6. transferring the content to a 500-mL bottle and add an equal volume of isopropyl alcohol;
7. agitating vigorously for 5 minutes and immediately centrifuge at 2,000 rpm at 20°C for approximately 10 minutes, using a JA14 rotor and a Beckham floor centrifuge, Model Avanto J251;
8. eliminating the supernatant and wash with an alcohol solution at 50%, in the same conditions specified in step 7;
9. re-suspending the precipitated material in a mixture of polysorbate 80-water (1:20) and maintain at -40°C for at least 12 hours; and
10. liofilizing in a Labconco apparatus at 1.33 x 10⁻¹ millibars and -46°C for 72 hours.

According to the methods disclosed in this description, a sustained drug release system for ophthalmic use, which is prepared based on biodegradable microspheres of polymers or matrixes is obtained, said system comprising:
a) a viscoelastic solution deriving from HA at 1.6% (Biovisc^{®});
b) a steroid drug for anti-inflammatory and immunosuppressant use, therapeutically acceptable for the treatment of ocular ailments with a final minimum concentration of 10 mM, or quinalone, non-steroid anti-inflammatory, immunosuppressant or drug that is compatible with the incorporation system; and
c) a chitosan solution at 2% in diluted acetic acid.

Although the invention, matrices, microspheres and methods for the preparation thereof have been described in their preferred realization, it shall be understood that some other modifications shall be deducible for an expert in the field, in light of that described hereinabove. Accordingly, the right of any possible modification is claimed, which in light of the above information could come about in the understanding that the scope of the claimed protection shall be defined by the contents of the following claims.

## Claims

1. An implant for sustained drug release for ophthalmic use, which is prepared with biodegradable microspheres of polymers or matrices, comprising:
a) a viscoelastic solution deriving from hyaluronic acid (HA) at 1.6%;
b) a steroid drug for anti-inflammatory and immunosuppressant use, therapeutically acceptable for the treatment of ocular ailments having a final minimum concentration of 10 mM, or quinolone, non-steroid anti-inflammatory, immunosuppressant or a drug that is compatible with the incorporation system; and
c) a chitosan solution at 2% in diluted acetic acid.

2. The implant of sustained drug release for ophthalmic use in accordance with claim 1, in which the viscoelastic solution comprises: HA at 1.6%, sodium chloride at 0.88%, dibasic phosphate of anhydrous sodium at 0.058%, monobasic phosphate of monohydrated sodium at 0.0126% and sterile water for injection as a base for 100%.

3. The implant of sustained drug release for ophthalmic use in accordance with claim 1, in which the viscoelastic solution has a dynamic viscosity of 35,000 ± 5,000 mPas, an osmolarity between 200 and 400 mOsm and a pH between 6.8 and 7.6.

4. The implant of sustained drug release for ophthalmic use in accordance with claim 1, in which the viscoelastic solution is Biovisc^{®}.

5. The implant of sustained drug release for ophthalmic use in accordance with claim 1, in which the therapeutically acceptable drug is dexamethasone.

6. The implant of sustained drug release for ophthalmic use in accordance with claim 1, in which the therapeutically acceptable drug is selected from the group consisting of any other steroid, quinolone, a non-steroid anti-inflammatory, an immunosuppressant or a drug that is compatible and stable with the aforementioned system.

7. Microspheres as a system of sustained drug release for ophthalmic use, comprising:
a) a viscoelastic solution deriving from HA at 1.6%;
b) a steroid drug for anti-inflammatory and immunosuppressant use, therapeutically acceptable for the treatment of ocular ailments with a final minimum concentration of 10 mM, or quinolone, a non-steroid anti-inflammatory, an immunosuppressant or a drug that is compatible with the incorporation system; and
c) a chitosan solution at 2% in diluted acetic acid.

8. The microspheres as a system of sustained drug release for ophthalmic use in accordance with claim 7, in which the viscoelastic solution is Biovisc^{®}.

9. The microspheres as a system of sustained drug release for ophthalmic use in accordance with claim 7, in which the therapeutically acceptable drug is dexamethasone.

10. A system of sustained release of drugs for ophthalmic use in accordance with claim 7, in which the therapeutically acceptable drug is selected from the group consisting of any other steroid, quinolone, a non-steroid anti-inflammatory, an immunosuppressant or a drug that is compatible and stable with the aforementioned system.

11. A method of preparing a matrix of biodegradable polymers comprising the following steps:
a) diluting the exact volume of the solution deriving from HA at 1.6% (Biovisc^{®}) in bidistilled water in order to obtain a final concentration of 0.025%;
b) adding to the above diluted solution, the exact amount of a solution of 100 mM of dexamethasone phosphate at a final concentration of 30 mM;
c) mixing to homogeneity with a Caframo^{®} agitator, using a double-blade propeller inverted at 45° with a diameter of 5 cm;
d) adjusting the pH, if necessary, to around 4.8 using hydrochloric acid 0.1 N;
e) agitating for approximately 1 hour at 100 revolutions per minute;
f) adding the exact amount of a solution of chitosan of crab shells at 2% dissolved in acetic acid in order to obtain a final concentration of 0.8%;
g) agitating, for approximately 1 hour, all the components added in the above steps, as specified in step e, until a gel-like insoluble solution is formed;
h) centrifuging the product of the preceding step at 1,500 rpm for approximately 15 minutes;
i) eliminating the supernatant and rinse the solid sediment with a solution of sodium chloride and phosphates pH 7.0;
j) washing three times by the same buffer in order to eliminate the free dexamethasone phosphate;
k) transferring the wet solid matter to a wide-neck bottle and maintain at - 40°C for at least 12 hours; and
l) liofilizing in a Labconco apparatus at 1.33 x 10⁻¹ millibars and -46°C for 72 hours.

12. A biodegradable polymer matrix for the preparation of implants of sustained drug release for ophthalmic use which is obtained by means of the method of claim 11.

13. A method of preparing biodegradable polymer microspheres, comprising the following steps:
a) mixing, in a first recipient, 6.25 mL of the Biovisc^{®} solution, with 12 mL of dexamethasone phosphate 100 mM for approximately 15 minutes at about 150 rpm until homogeneity is achieved;
b) mixing, in a second recipient, 74.4 mL of liquid paraffin (mineral oil) with 1.6 mL of Span 80, for approximately 15 at about 150 rpm until homogeneity is achieved;
c) slowly pouring the contents of the first recipient into the second recipient, while maintaining the same agitation speed, until both contents are incorporated, and increase the centrifuging speed to 1,300 rpm and agitate for at least 12 hours;
d) adding 21.75 mL of chitosan of crab shells at 1.47% until the full incorporation thereof into the product obtained from the above step;
e) agitating the content of the recipient in the conditions specified in step c), for 24 hours;
f) transferring the content to a 500-mL bottle and add an equal volume of isopropyl alcohol;
g) agitating vigorously for approximately 5 minutes and immediately centrifuge at 2,000 rpm at 20°C for around 10 minutes;
h) eliminating the supernatant and wash with an alcohol solution at 50%, in the same conditions specified in step g);
i) re-suspending the precipitated material in a mixture of polysorbate 80-water (1:20) and maintain at -40°C; and
j) liofilizing the resulting product at 1.33 x 10⁻¹ millibars for 72 hours.

14. The biodegradable polymer microspheres as a system of sustained drug release for ophthalmic use, which are obtained by means of the method of claim 13.
